# EUROPEAN PATENT APPLICATION

(11) **EP 4 817 550 A1**
(43) Date of publication of application: **07.10.2026**
(21) Application number: 25168302.5
(22) Date of filing: 03.04.2025
(51) Int. Cl.: A61B 34/30, A61F 9/007, A61B 90/00, A61B 34/10

(54) **SURGICAL ROBOTIC SYSTEM COMPRISING EYE MANIPULATION SYSTEM**

(71) Applicant: Carl Zeiss Meditec AG, 07745 Jena (DE)
(72) Inventor: MEENINK, Hildebert Christiaan Matthijs, 5656 AG Eindhoven (NL); BEELEN, Maarten Joannes, 5656 AG Eindhoven (NL); KIENLE, Patrick, 81379 München (DE)
(74) Representative: DeltaPatents B.V.

(57) **Abstract**

A surgical robotic system for ophthalmic surgery, comprising an instrument manipulator (240) for mounting a surgical instrument (250), wherein the surgical robotic system is configured to establish a remote center of motion, RCM, for the surgical instrument and to control a position of the RCM in three-dimensional space, wherein the surgical robotic system comprises a processor subsystem configured to reposition the RCM to track the entry point during orbital manipulation of the eye by accessing a model indicative of a repositioning of the entry point in three-dimensional space during orbital manipulation of the eye; repositioning the RCM in accordance with the model during the orbital manipulation of the eye; obtaining intraoperative sensor data and analyzing the intraoperative sensor data to detect a deviation of the RCM from the entry point and/or the ocular surface of the eye; and adjusting the position of the RCM based on the detected deviation. This may reduce the reliance on a perfectly accurate model, while still enabling the RCM to track the entry point during orbital manipulation.

## Description

### TECHNICAL FIELD

The presently disclosed subject matter relates to surgical robotic system for ophthalmic surgery.

### BACKGROUND

Ophthalmic surgical procedures increasingly involve the use of surgical robotic systems. Rather than operating entirely autonomously, such surgical robotic systems are expected to remain in the near future at least in part under the control of a human operator. For example, the human operator may directly or indirectly control the movement of a surgical instrument mounted to a surgical arm of the surgical robotic system. Nevertheless, it is expected that future surgical robotic systems may be more autonomous and require less or even no involvement of a human operator.

A surgical robotic system designed for ophthalmic surgical procedures may be provided with an instrument manipulator for mounting a surgical instrument. The instrument manipulator may have at least one degree of freedom to enable adjustment of a pose of the surgical instrument. An actuation subsystem may be provided which may be arranged to effect the adjustment of the pose of the surgical instrument. Here, the term 'pose' may refer to a position and orientation of the surgical instrument. Through such actuation, the surgical instrument may for example be moved in a lateral direction, for example relative to an anatomical surface such as a retinal surface, and/or in a longitudinal direction along a longitudinal axis of the surgical instrument. Longitudinal movement may typically allow the surgical instrument to be moved towards and away from a surgical target. Accordingly, the surgical instrument may be used to modify biological tissue near the surgical target, to deliver an agent to the surgical target, etc. Examples of surgical instruments include, but are not limited to, forceps, cutters, coagulation cutters, scissors, injection needles, sealing devices, etc.

While the use of surgical robotic systems in ophthalmic surgical procedures offers numerous benefits, including enhanced precision and dexterity, it may be required that the pose of the eye relative to the surgical instrument is known during surgery so that the pose of the surgical instrument may be appropriately controlled. Here, the term 'pose of the eye' may include the position and orientation of the eye. The position of the eye may be determined by or based on a position of the head of a patient. The orientation of the eye may be determined by the orientation of the head and by the orientation of the eye within the eye socket. For example, during intraocular surgery, the head of the patient may be brought into a known position and orientation and the orientation of the eye within the eye socket may be fixated. For example, it is known to establish a coupling between the surgical robotic system and the eye by using a cannula interface to couple with a cannula inserted into the eye, thereby fixating the orientation of the eye with respect to the surgical robotic system.

In addition to the static fixation of the eye, there may also be a need to manipulate the orientation of the eye during an ophthalmic surgical procedure. Such manipulation may involve rotating the eye within the eye socket and thereby changing the orientation of the eye with respect to one or more external surgical entities, such as the instrument manipulator of a surgical robotic system, a microscope, etc. The externally induced change in the orientation of the eye may also be referred to as orbital manipulation since it involves altering the eye's orientation within the orbital cavity. Orbital manipulation may be advantageous for various reasons, including but not limited to improving the visualization or observability of peripheral regions of the eye and enhancing the reach of surgical instruments within the eye.

It is known to manually perform orbital manipulation. For example, a surgeon may rotate the eye using two instruments that are inserted into the eye.

There also exist robotic systems that can perform orbital manipulation of the eye. For example, the paper "Vitreoretinal Surgical Robotic System with Autonomous Orbital Manipulation using Vector-Field Inequalities" by Koyama et al., 2023, https://arxiv.org/abs/2302.05567, describes autonomous orbital manipulation of the eye in robot-assisted vitreoretinal surgery, in which a surgical needle is used as a first surgical instrument and a light guide as a second surgical instrument to jointly rotate the eye within the eye socket. The paper is concerned with determining a control strategy to appropriately control both surgical instruments to manipulate the eye.

Disadvantageously, existing robotic systems for orbital manipulation may risk damaging the eye, for example with an inserted surgical instrument if the pose of the surgical instrument is not properly controlled during eye rotation.

### SUMMARY

An aspect of the presently disclosed subject matter provides a surgical robotic system for ophthalmic surgery, comprising:
- an instrument manipulator for mounting a surgical instrument, wherein the surgical instrument comprises an intraocular portion for insertion into an eye via an entry point on an ocular surface of the eye, wherein the instrument manipulator comprises at least one degree of freedom to enable adjustment of a pose of the surgical instrument;
- an actuation subsystem configured to actuate the adjustment of the pose of the surgical instrument; and
- a processor subsystem configured to control the actuation subsystem to control the adjustment of the pose of the surgical instrument;

wherein the surgical robotic system is configured to establish a remote center of motion, RCM, for the surgical instrument and to control a position of the RCM in three-dimensional space;
wherein the processor subsystem is configured to reposition the RCM to track the entry point during orbital manipulation of the eye by:
   - accessing a model indicative of a repositioning of the entry point in three-dimensional space during orbital manipulation of the eye;
   - repositioning the RCM in accordance with the model during the orbital manipulation of the eye;
   - obtaining intraoperative sensor data and analyzing the intraoperative sensor data to detect a deviation of the RCM from the entry point and/or the ocular surface of the eye; and
   - adjusting the position of the RCM based on the detected deviation.

The above aspect of the invention involves providing a surgical robotic system which comprises an instrument manipulator for mounting a surgical instrument. Instrument manipulators for surgical robotic systems are known per se in the field of medical robotics, and may take over tasks of a surgeon's hand, for example by handling and manipulating a surgical instrument. A surgical instrument may be a tool or device for performing actions to carry out desired effects during a surgery or operation, such as modifying biological tissue or providing access to observe the biological tissue. Examples of surgical instruments include, but are not limited to, forceps, mechanical cutters, coagulation cutters, scissors, injection needles, sealing devices, etc.

The instrument manipulator may have at least one degree of freedom (DoF) to enable a pose of the surgical instrument to be adjusted. Here, the term 'pose' may refer to the position and/or orientation of the surgical instrument. As such, using the at least one DoF of the instrument manipulator, the position and/or orientation of the surgical instrument may be adjusted. To provide the instrument manipulator with the at least one DoF, the instrument manipulator may for example be mounted to a surgical arm. For example, the surgical arm may comprise a linkage system to which the instrument manipulator may be movably suspended, such as a parallelogram linkage mechanism. The linkage system may be connected to a base. It is noted that the instrument manipulator may also be provided with the at least one DoF using other mechanisms besides a surgical arm, such as linear actuators, a gantry system, etc.

The surgical instrument may typically have a longitudinal axis which may typically pass through a tip of the surgical instrument. The at least one DoF may enable the tip of the surgical instrument to be moved, for example laterally in relation to an anatomical surface, such as a retinal surface or the surface of another structure within or on the eye. Typically, the instrument manipulator may be provided with three or more DoFs, for example to enable longitudinal movement of the surgical instrument along the longitudinal axis, for example to advance towards and/or retract away from a surgical target, and to enable lateral movement of the tip of the surgical instrument along a plane perpendicular to the longitudinal axis. It is noted that instrument manipulators having the functionality described in this paragraph and in the preceding paragraph may be known per se from the field of medical robotics.

An actuation subsystem may be provided for effecting the adjustment of the pose of the surgical instrument. For example, the actuation subsystem may comprise one or more actuators to actuate the instrument manipulator and/or to actuate components through which the instrument manipulator is movably suspended, such as the aforementioned surgical arm, such as the parallelogram linkage system, and/or the base. Another term for actuation subsystem may be 'driving mechanism'.

The surgical robotic system may be configured to establish a Remote centre of Motion (RCM) for the surgical instrument and to control the position of the RCM in three-dimensional space. As is known per se, an RCM may constrain movements of the surgical instrument such that its trajectories intersect the RCM in three-dimensional space. Typically, the RCM lies along the longitudinal axis of the surgical instrument and may be positioned at an entry point to the eye, for example at or near a cannula providing an opening into the eye. By positioning the RCM at the entry point, the surgical instrument may be adjusted in pose, for example by advancing, retracting, or moving within the eye while pivoting about the RCM. The RCM may help prevent the surgical instrument from exerting unnecessary stress on the eye at the entry point.

The RCM may be established by the surgical robotic system in several ways. For example, the RCM may be enforced through software control, ensuring that, regardless of actuation of the instrument manipulator, the surgical instrument remains constrained to movements that comply with the RCM. Additionally, or alternatively, the surgical robotic system may be mechanically designed to establish an RCM. A combination of software and hardware constraints is also possible, where certain degrees of motion are mechanically restricted while others are controlled through software. The RCM may be variable in that the RCM may be repositioned along the surface of the eye, typically through actuation. Such actuation may be performed using the same actuators that control the pose of the surgical instrument. Alternatively, the RCM may be repositioned using one or more dedicated actuators. Accordingly, in some examples, the actuation subsystem described in this specification may include actuators specifically configured to reposition the RCM of the surgical instrument.

The repositioning of the RCM may be controlled by the processor subsystem, for example by generating and executing appropriate control commands. The inventors have recognized that if orbital manipulation is performed during an ophthalmic procedure, the RCM may need to be repositioned along with the entry point via which the surgical instrument accesses the interior of the eye. For example, when the surgical instrument is inserted into the eye and orbital manipulation is performed using a separate eye manipulation device, such as a vacuum cup, failing to reposition the RCM, and consequently failing to reposition the surgical instrument at the entry point along with the eye, could cause damage at the entry point. This is because the surgical instrument may constrain the eye's natural rotational movement, counteracting the force applied by the eye manipulation device and potentially creating excessive stress at the entry site. Moreover, when the surgical instrument itself is used for eye manipulation, as for example described by Koyama et al., force may be most effectively applied at the entry point, which may necessitate a coordinated movement of the entry point and the RCM to ensure effective transmission of force onto the eye. Even when the surgical instrument is not inserted into the eye, tracking the entry point with the RCM remains advantageous. For example, tracking may allow the surgical robotic system to maintain alignment with the eye if the surgical robotic system interfaces with the eye through another means, such as a cannula interface. Such tracking may also ensure that the surgical robotic system is already correctly positioned when the surgical instrument is to be inserted, which may improve procedural efficiency.

The above measures involve accessing a model that is indicative of the repositioning of the entry point in three-dimensional space during orbital manipulation of the eye. For example, the model may be a spherical model, which may indicate that the movement of the entry point during orbital manipulation resembles movement of a point along the surface of a sphere. The processor subsystem may then reposition the RCM in accordance with the model during orbital manipulation of the eye. Such repositioning of the RCM may involve tracking the entry point to ensure that the surgical instrument remains properly aligned with the entry point. For that purpose, the processor subsystem may obtain orbital manipulation data which characterizes the orbital manipulation of the eye and using the orbital manipulation data together with the model to determine or estimate the movement of the entry point and adjust the position of the RCM accordingly. For example, the orbital manipulation data may characterize the rotation of the eye in terms of angular displacement, and based on the model, the processor subsystem may determine the coordinates of the entry point in three-dimensional space during the orbital manipulation. Such orbital manipulation data may be obtained internally, e.g., when the surgical robotic system itself carries out the orbital manipulation, or externally, e.g., from an eye manipulation system carrying out the orbital manipulation or from sensor data indicative of the orbital manipulation.

The inventors have recognized that such modeling may be imperfect. For example, when assuming a spherical model, as in Koyama et al., the modeling may fail to account for deviations from an idealized spherical eye shape. Moreover, even if the model were perfectly accurate, practical limitations in positioning the RCM according to the model may arise due to control inaccuracies and system imperfections. The inventors have further recognized that deviations resulting from imperfect modeling or inaccuracies in applying the model may be detected during the ophthalmic procedure from intraoperative sensor data, and that the position of the RCM may then be corrected based on the detected deviation, for example to reduce the deviation.

Accordingly, the surgical robotic system is configured to obtain intraoperative sensor data, referring to sensor data acquired during the ophthalmic procedure, and to analyze the intraoperative sensor data to detect a deviation of the RCM from the entry point and/or the ocular surface of the eye and take appropriate corrective action... This reduces reliance on a perfectly accurate model, which may be impractical to obtain in real-world conditions, while still enabling the RCM to effectively track the entry point during orbital manipulation. Accordingly, by combining model-based estimation with intraoperative sensor data, the surgical robotic system can compensate for model inaccuracies, control inaccuracies, and system imperfections.

While in principle it may be possible to reposition the RCM purely using a feedback control system based on intraoperative sensor data, the combined use of a model with intraoperative sensor data allows the surgical robotic system to anticipate the expected movement of the entry point, enabling a combination of predictive adjustments and real-time corrections. This approach may reduce the likelihood of significant deviations that would otherwise require large corrective actions. Moreover, relying solely on intraoperative sensor data for repositioning may lead to delayed responses, as adjustments could only be made after deviations occur.

Optionally, the surgical robotic system is configured to effect the orbital manipulation of the eye by engaging and applying a force to the eye to reorient the eye within an eye socket. In some examples, the surgical robotic system may be configured to perform the orbital manipulation of the eye itself. This may be achieved using known techniques, such as a vacuum cup that engages the eye and is controlled by one or more actuators to apply a force for reorienting the eye within the eye socket. In such cases, data related to the orbital manipulation may be available internally within the surgical robotic system, allowing the processor subsystem to determine how the orbital manipulation proceeds and adjust the RCM accordingly. Additionally, in some examples, the repositioning of the RCM and the orbital manipulation may be jointly controlled. For instance, the processor subsystem may implement an integrated control algorithm that coordinates both the repositioning of the RCM and the orbital manipulation to effect the desired orbital manipulation of the eye while maintaining proper alignment of the surgical instrument at the entry point.

Optionally, the instrument manipulator comprises a cannula interface configured to releasably engage a cannula inserted into the eye at the entry point, and wherein the processor subsystem is configured to at least in part effect the orbital manipulation of the eye by applying the force with the cannula interface to the cannula. The surgical robotic system may be configured to releasably engage a cannula inserted into the eye, for example through a mechanical, magnetic, or other releasable mechanism, also referred to as a cannula interface. The cannula interface may be affixed to a distal end of the instrument manipulator. By engaging the cannula, the surgical robotic system may establish a consistent reference point for subsequent manipulations, allowing precise control of the surgical instrument and the forces applied to the eye. The inventors have recognized that the surgical robotic system may at least in part effect the orbital manipulation of the eye by applying a force to the cannula via the cannula interface, causing the force to be transmitted to the eye and causing the eye to rotate with the eye socket. Here, 'at least in part' may denote that in some examples, the forces required for orbital manipulation may be provided not only through the cannula interface but also through additional mechanisms. The use of a cannula interface for orbital manipulation may be advantageous, as the cannula interface may already be integrated into the surgical robotic system for surgical instrument guidance, reducing the need for a separate eye manipulation device.

Optionally, the surgical instrument is inserted through a cannula into the eye, wherein the processor subsystem is configured to at least in part effect the orbital manipulation of the eye by applying the force with the inserted instrument to the cannula. As is known per se from manual orbital manipulation, orbital manipulation may be performed using an inserted instrument. The surgical robotic system may at least in part effect orbital manipulation in this manner. For this purpose, the processor subsystem may be configured to apply force with an inserted surgical instrument to the cannula, thereby transmitting the force to the eye to induce rotation within the eye socket. This approach may be advantageous since the surgical instrument is already present for the procedure, eliminating the need for a separate eye manipulation device. Additionally, it may obviate the need for the surgical robotic system to include a dedicated cannula interface, thereby simplifying the surgical robotic system's design.

Optionally, the processor subsystem is configured to at least in part effect the orbital manipulation of the eye by applying the force with the inserted instrument directly to the eye, for example to the cornea or sclera adjacent to the entry point. It is conceivable to omit a cannula during an ophthalmic procedure. In this case, the force exerted by the surgical robotic system using the surgical instrument may be applied directly to the eye, for example to the cornea or the sclera adjacent to the entry point.

Optionally, the model is configured to provide a modeled representation of the ocular surface of the eye. The model used by the processor subsystem may specifically represent the ocular surface of the eye, including its curvature. The curvature may provide information to the processor subsystem on how a point on the surface of the eye, such as the entry point, moves in three-dimensional space as the eye rotates. By incorporating such a model, the processor subsystem may effectively determine the movement of the entry point during orbital manipulation.

Optionally, the processor subsystem is configured to adapt the model to a patient-specific shape of the eye based on an estimate of an ocular radius of the eye. The inventors have recognized that interpatient variability in eye shape is for a significant part determined by differences in ocular radius. By estimating the ocular radius, the processor subsystem may adapt the model to more accurately reflect the patient-specific shape of the eye, which in turn may improve the accuracy of determining the movement of the entry point during orbital manipulation.

Optionally, the model is a parameterized geometric model, and wherein the processor subsystem is configured to determine a center of rotation and/or spatial dimensions of the geometric model based on the estimate of the ocular radius of the eye. The processor subsystem may be configured to use a geometric model of the eye, such as a spherical or ellipsoidal model, to internally represent the ocular surface. Using an ellipsoidal model may be advantageous, as it provides a more realistic representation of the eye, which is typically ellipsoid in shape. The geometric model may be initialized based on the estimate of the ocular radius, allowing the processor subsystem to determine key parameters such as the center of rotation, which may be located within the interior of the eye based on the estimated ocular radius, as well as the overall spatial dimensions of the eye. This may allow the processor subsystem to adapt the model to more accurately reflect the patient-specific shape of the eye.

Optionally, the processor subsystem is configured to adapt the model to the patient-specific shape of the eye using extraocular sensor data indicative of a cross-sectional profile or curvature of the eye and/or using intraocular sensor data indicative of a distance between the inserted surgical instrument and the retinal surface. The model may be adapted to the patient-specific shape of the eye based on sensor data, which may be obtained intraoperatively during the ophthalmic procedure or preoperatively. For example, extraocular sensor data may be acquired by one or more sensors positioned outside the eye. Such sensor data may be indicative of the curvature of the eye, for example by measuring the ocular surface from outside of the eye or by generating a cross-sectional profile of the eye, as in the case of optical coherence tomography (OCT). Additionally, or alternatively, the processor subsystem may make use of intraocular sensor data, which may be obtained from a sensor affixed to the surgical instrument. Such intraoperative sensor data may be indicative of the distance between the inserted surgical instrument and the retinal surface. This distance measurement may provide information about the patient-specific shape of the eye from within, which may in turn be predictive of the outer surface curvature of the eye.

Optionally, the processor subsystem is configured to:
- obtain a distance measurement from a distance sensor, wherein the distance data is indicative of a distance between the surgical instrument and an ocular structure;
- adjust the pose of the surgical instrument to obtain a plurality of distance measurements from the distance sensor; and
- update the model based on the plurality of distance measurements.

The processor subsystem may actively determine the patient-specific shape of the eye using a distance sensor. The distance sensor may provide distance data indicative of a distance between the surgical instrument and an ocular structure, such as an intraocular structure (e.g., the retina) or an extraocular structure (e.g., the sclera). The processor subsystem may adjust the pose of the surgical instrument to measure distances between the surgical instrument and the ocular structure at multiple positions relative to one another. The pose of the surgical instrument may be generally known to the processor subsystem since the pose may be controlled by the processor subsystem. Therefore, a plurality of such measurements, when correlated with the known pose of the surgical instrument. may provide a spatial mapping of the ocular structure, for example the retinal surface. Based on this mapping, the processor subsystem may update the model accordingly, for example by fitting the model to data points representing, or having been derived from, the plurality of distance measurements. In some examples, the distance sensor may be an instrument-attached distance sensor, in that at least part of the distance sensor may be affixed to an intraocular portion of the surgical instrument. In other examples, the distance sensor may be an extraocular distance sensor, e.g., an extraocular OCT sensor.

Optionally, the processor subsystem is configured to analyze the intraoperative sensor data to detect a deviation between the modeled representation of the ocular surface of the eye and the actual ocular surface of the eye, and to update the model based on the detected deviation. The intraoperative sensor data may not only indicate a deviation between the RCM and the entry point and/or the ocular surface but may also indicate inaccuracies in the model itself. Similar to how deviations between the RCM and the entry point and/or the ocular surface may be corrected, the processor subsystem may update the model to correct for such inaccuracies in the modelling. This may allow the processor subsystem to refine the model dynamically during use, for example in a gradual or iterative manner. For example, the model may initially be a generic spherical or ellipsoidal representation of the eye, which is then progressively adapted to the patient-specific shape based on intraoperative sensor data. The processor subsystem may achieve this by continuously, periodically, or at least repeatedly adjusting parameters of the model in response to detected deviations between the modeled representation of the ocular surface and the actual ocular surface. By improving the model in real time, the accuracy of tracking the entry point by repositioning the RCM may be improved.

Optionally, the processor subsystem is configured to update the model based on the detected deviation of the RCM from the entry point and/or the ocular surface of the eye. The deviation of the RCM from the entry point and/or the ocular surface may be indictive of the aforementioned deviation between the modeled representation of the ocular surface of the eye and the actual ocular surface of the eye. As such, the processor subsystem may, in response to a detected deviation, adjust certain parameters of the model, such as an estimate of the radius of the eye. For example, the adjustment may be performed in small stepwise increments, allowing the model to be refined progressively through repeated updates, thereby optimizing the representation of the ocular surface over time.

Optionally, the intraoperative sensor data comprises image data of the entry point, for example acquired using a microscope, wherein the processor subsystem is configured to analyze the image data to detect a deviation of the RCM from the entry point and/or the ocular surface, for example by detecting an indentation of the eye caused by the surgical robotic system. Image data may indicate a deviation between the RCM and the ocular surface and/or the entry point. Such image data be acquired extraocularly, for example using a microscope. The RCM may be identified in the image data based on its known position. For example, the RCM may be generally aligned with the surgical instrument or co-located with a cannula interface. The processor subsystem may compare this position with the detected ocular surface and/or the detected entry point to determine any deviation of the RCM from the entry point and/or the ocular surface. If a deviation is found, the system may apply a correction. In some cases, an inaccurate model may cause the surgical robotic system to indent the eye, for example with a cannula interface or another component. Such indentation may indicate that the RCM is positioned too close to the eye, prompting the system to retract the RCM. Interestingly, this type of deviation may be detected even without explicitly determining the exact positions of the RCM and the ocular surface. In some cases, an inaccurate model may cause the cannula interface to lose contact with the cannula at the entry point. This type of deviation may be detected by analyzing intraoperative sensor data to identify the position of the cannula and comparing the detected position to the position of the cannula interface, which is typically co-located with the RCM. By detecting such a deviation, the processor subsystem may adjust the position of the RCM to restore proper alignment between the cannula interface and the cannula at the entry point.

Optionally, the intraoperative sensor data comprises intraocular pressure data acquired from an intraocular pressure sensor, wherein the processor subsystem is configured to adjust the position of the RCM based on a change in measured intraocular pressure. A rise in intraocular pressure may indicate that the RCM is too close to the eye. This may cause indentation or excessive force on the eye by components of the surgical robotic system, leading to an increase in intraocular pressure. Likewise, a drop in intraocular pressure may indicate that the RCM is too far from the eye. The processor subsystem may adjust the position of the RCM accordingly to maintain proper alignment and avoid unnecessary stress on the eye.

Optionally, the intraoperative sensor data comprises force sensor data indicative of a force exerted on the eye by the surgical robotic system, wherein the processor subsystem is configured to adjust the position of the RCM based on a change in measured force. The force required for orbital manipulation may be expected to fall within a certain range. Deviations outside this range may indicate that the RCM is not correctly positioned relative to the ocular surface. For example, an increase in measured force may suggest that the RCM is too close to the eye, causing components of the surgical robotic system, such as a cannula interface, to apply force inefficiently rather than directly contributing to eye rotation. Conversely, a drop in measured force may indicate that the RCM is too far from the eye, leading to a loss of contact between the surgical robotic system and the ocular surface. The processor subsystem may adjust the RCM position accordingly to maintain proper alignment and ensure effective force transmission while avoiding unnecessary stress on the eye.

Optionally, the force data is obtained from a force sensor attached to the instrument manipulator, wherein the force sensor is configured to measure a force exerted by a cannula interface attached to the instrument manipulator to a cannula inserted into the eye and/or a force exerted by the inserted surgical instrument to the cannula or directly to the eye. Forces may be measured by a force sensor attached to the instrument manipulator. Such a force sensor provides an effective way to obtain sensory feedback, allowing the processor subsystem to monitor and adjust the applied forces to maintain proper alignment and avoid unnecessary stress on ocular tissue.

Optionally, the processor subsystem is configured to adjust the position of the RCM based on the intraoperative sensor data by:
- retracting the RCM away from the ocular surface if a measured parameter of the intraoperative sensor data exceeds an upper threshold or if an increase in the measured parameter exceeds an upper change threshold; and/or
- advancing the RCM toward the ocular surface if the measured parameter falls below a lower threshold or if a decrease in the measured parameter exceeds a lower change threshold.

Optionally, the intraoperative sensor data comprises distance data from a distance sensor, wherein the processor subsystem is configured to adjust the position of the RCM based on a change in measured distance. For example, the distance sensor may be an instrument-attached distance sensor or an extraocular distance sensor, e.g., an extraocular OCT sensor. It may be expected that a correct repositioning of the RCM results in the internal distance between the surgical instrument and a reference structure, such as the retinal surface, remaining relatively constant. Conversely, a change in this measured distance during orbital manipulation, without an intentional repositioning of the instrument, may indicate an unintentional shift in the relative position of the RCM with respect to the ocular surface. The processor subsystem may adjust the RCM position accordingly to counteract changes in the measured distance and maintain proper alignment.

Optionally, the processor subsystem is configured to adjust the pose of the surgical instrument during the repositioning of the RCM to maintain a constant relative pose of the surgical instrument with respect to a retinal surface of the eye. If the surgical instrument is inserted into the eye, the repositioning of the RCM may be accompanied by an adjustment of the pose of the surgical instrument to ensure that its relative pose, such as its position and/or orientation, with respect to the retinal surface remains relatively constant. Maintaining a consistent relative pose may be desirable for predictability and procedural continuity, allowing the surgical procedure to resume from the same relative pose after orbital manipulation as before the orbital manipulation. In addition, maintaining a constant relative pose may help prevent unintended movements of the surgical instrument that could otherwise cause damage within the eye. Alternatively, it may be desirable to adjust the pose of the surgical instrument such that the surgical instrument remains visible to the surgeon through a microscope during the procedure. For that purpose, the processor subsystem may access and analyze intraoperative microscope data, which may provide real-time feedback on the visibility of the surgical instrument. In some implementations, the processor subsystem may adjust the instrument pose based on an assumed location within the eye, ensuring that the instrument remains within the field of view. By dynamically adjusting the pose of the surgical instrument according to visibility constraints, the processor subsystem may maintain continuous visualization of the instrument throughout the procedure.

Optionally, the processor subsystem is configured to:
- obtain data indicative of an initial orientation of the eye relative to the instrument manipulator prior to orbital manipulation; and
- account for the initial orientation of the eye when applying the model for repositioning the RCM.

The initial orientation of the eye may be used to rotationally align the model with the eye's actual orientation relative to the instrument manipulator. This may be particularly relevant when both the actual eye shape and its modeled representation deviate from a perfect sphere. For example, if the model is an ellipsoidal representation of the eye, its orientation may be adjusted to match the initial orientation of the eye, ensuring consistency between the model and the actual anatomy. Data on the initial orientation may be obtained from, for example, sensor data, provided by a user, or derived through other means. Aligning the model in this manner may improve the accuracy of repositioning the RCM during orbital manipulation.

Optionally, the RCM is positioned at the entry point prior to orbital manipulation, and wherein the processor subsystem is configured to:
- obtain data indicative of the position of the entry point on the eye;
- use the position of the entry point as an initial position of the RCM when using the model for repositioning the RCM.

The actual position of the entry point relative to the eye may serve as a reference for determining an initial position on the ocular surface as represented by the model. This initial position may then be used as a starting point for predicting the movement of the entry point over the ocular surface during orbital manipulation. By obtaining data indicative of the entry point position and aligning the model accordingly, the processor subsystem may improve the accuracy of repositioning the RCM.

It will be appreciated by those skilled in the art that two or more of the above-mentioned embodiments, implementations, and/or aspects of the presently disclosed subject matter may be combined in any way deemed useful.

### BRIEF DESCRIPTION OF THE DRAWINGS

These and other aspects of the presently disclosed subject matter are apparent from and will be elucidated with reference to the embodiments described hereinafter. In the drawings,
Fig. 1 shows a schematic representation of a surgical robotic system;
Fig. 2A schematically shows a field of view when viewing the eye straight ahead, which field of view encompasses the central posterior region of the eye;
Fig. 2B schematically shows a field of view obtained by rotating the eyeball within the eye socket, which field of view includes a peripheral posterior region of the eye;
Fig. 3 shows a sideways orientation of the eye during gonioscopy to, for example, visualize the trabecular meshwork using a gonio prism;
Fig. 4 shows a cannula interface of a surgical robotic system to, during a surgical procedure, engage a cannula inserted into the eye;
Fig. 5 shows the surgical robotic system comprising an instrument manipulator for holding a surgical instrument, wherein the surgical robotic system is configured to establish a variable remote center of motion of the surgical instrument;
Figs. 6A and 6B schematically illustrate orbital manipulation of the eye by the surgical robotic system of Fig. 5 using the cannula interface, wherein both the cannula interface and the remote center of motion are repositioned simultaneously;
Fig. 7 illustrates the use of models that inaccurately represent the radius and center of rotation of the eye, which may result in the cannula interface becoming loose or detached from the cannula, or causing the cannula to indent the eye;
Fig. 8 shows a top-down anterior view of the eye, showing various cannula placements relative to a clock face reference;

It should be noted that items which have the same reference numbers in different figures, have the same structural features and the same functions, or are the same signals. Where the function and/or structure of such an item has been explained, there is no necessity for repeated explanation thereof in the detailed description.

### List of reference numerals

The following list of references and abbreviations is provided for facilitating the interpretation of the drawings and shall not be construed as limiting the claims.
- 10: surgical robotic system
- 20: user interface subsystem
- 22: user inputs
- 30: sensor
- 32: sensor data
- 40: processor subsystem
- 42: actuation commands
- 60: actuator subsystem
- 62: actuation of surgical arm
- 80: surgical arm
- 82: movable arm part
- 100: eye
- 102: eyeball
- 104: eye socket
- 106: cornea
- 107: sclera
- 108: lens
- 110: field of view (central)
- 112: field of view (peripheral)
- 120: gonio prism
- 122: view axis
- 150: cannula
- 200: surgical robotic system
- 210: base
- 220: hold-and-release mechanism
- 230: (parallelogram) linkage system
- 240: instrument manipulator
- 250: surgical instrument
- 260-262: cannula interface
- 270: headrest
- 300: remote center of motion
- 310, 311: field of view
- 320, 321: visible part of retina
- 330-332: spherical eye model
- 340-342: center of rotation

### DESCRIPTION OF EMBODIMENTS

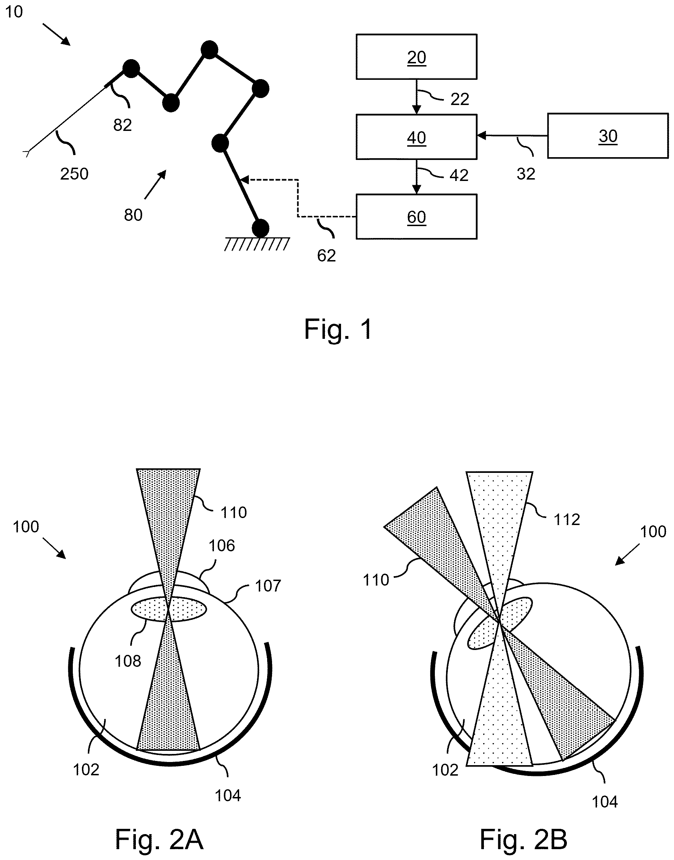

Fig. 1 schematically shows a surgical robotic system 10 for use in an intraocular surgical procedure. The surgical robotic system 10 may comprise a surgical arm 80. The surgical arm 80 may comprise a movable arm part 82 which may comprise an end effector (not explicitly shown in Fig. 1) for holding a surgical instrument 250. For example, the surgical instrument 250 may be an irrigation/aspiration instrument for irrigating or aspirating fluid at a desired target location, a photocoagulation instrument for applying optical energy at a desired distance from a target, a vitrectomy instrument for cutting and aspirating the vitreous humor or other fluidics at a desired distance from a target, a tissue manipulating instrument, which may need to be placed at the interface of a tissue, or the like. To hold the surgical instrument 250, the end effector may comprise an instrument connector to which the surgical instrument 250 may be mounted. The movable arm part 82 may have at least three degrees-of-freedom (DoF) to enable longitudinal movement of the surgical instrument towards a surgical target within the eye and lateral movement of the surgical instrument in a plane normal to said longitudinal movement. Here, longitudinal movement may refer to a movement of the surgical instrument 250 along its longitudinal axis (not separately shown in Fig. 1). In some embodiment, the surgical robotic system 10 may be configured to enable lateral movement in a curved plane. The curvature of the curved plane may for example follow a curvature of the interior of the eye. Such type of lateral movement along a curved plane may be obtained by the surgical robotic system adjusting the longitudinal position of the surgical instrument 250 during lateral movement. The shape of the curved plane may for example be determined based on a model of the eye or an intraocular structure, e.g., the retina.

In some embodiments, the surgical robotic system 10 may further comprise a user interface subsystem 20 for receiving user inputs 22 from a user. The user interface subsystem 20 may thus represent at least a user input interface, and in some embodiments, as also discussed elsewhere, additionally, or alternatively a user output interface. The user inputs 22 may for example comprise positioning instructions from a human operator, e.g., a surgeon, to enable the human operator to determine a trajectory of the surgical instrument. In some embodiments, the positioning instructions may be positioning commands for directly controlling the movement of the surgical instrument. In other embodiments, the positioning instructions may be provided as input during a planning procedure in which the trajectory of the surgical instrument is planned. Other types of user inputs may for example include confirmation inputs indicating instructions to continue with a procedure or procedural step, and/or input information, such as indicating an operating parameter and/or an indication of a surgical target position or the like. Examples of user input interfaces for receiving user inputs from a user include, but are not limited to, a keyboard, a mouse, a touch-sensitive surface, a joystick, a foot pedal, a microphone, a gesture recognition system, etc. The user input interface may employ any suitable input modality, such as touch, push-actions, voice commands, eye movements, gesture recognition, etc.

The surgical robotic system 10 may further comprise an actuator subsystem 60 configured and arranged for actuating the movable arm part to effect the longitudinal movement and lateral movement of the surgical instrument. The actuator subsystem 60 may comprise any suitable actuator(s), e.g., from the field of surgical robots, or from the more general field of actuators. In particular, the actuator subsystem 60 may comprise a plurality of actuators which together provide the actuation of the movable arm part 60 along the three or more DoFs. Accordingly, it will be appreciated that any reference to a specific configuration of the actuator subsystem 60 may be understood as referring to a (joint) configuration of such a plurality of actuators.

Fig. 1 shows the actuation of surgical arm 80 schematically, namely as a dashed line 62. It is noted that, although shown separately of the surgical arm 80, the actuator subsystem 60 may be integrated into, or mounted to, the surgical arm 80.

The surgical robotic system 10 may further comprise a sensor 30. The sensor 30 may be or comprise an optical coherence tomography (OCT) probe. The OCT probe may for example be an optical fiber which is attached to or integrated in the surgical instrument 250, with the optical fiber being connected to a remotely located OCT sensor. In other examples, the OCT probe may comprise an OCT sensor which is directly attached to or directly integrated in the surgical instrument 250. The sensor data 32 provided by the OCT probe may comprise A-scans, which may comprise line measurements and defined as an intensity as a function of the distance, B-scans forming a 2D image, C-scans, e.g., from multiple B-scans, or the like. It is noted that even though the sensor 30 is shown in Fig. 1 to be separate from the surgical instrument 250, the sensor or part of the sensor may be attached to or integrated in the surgical instrument 250. In other examples, the sensor 30 may, in addition or alternatively to the OCT probe, comprise a stereo camera arranged for image capture through a microscope, an optical interferometric sensor integrated in or attached to the surgical instrument 250, a time-of-flight sensor integrated in or attached to the surgical instrument and an ultrasonic sensor integrated in or attached to the surgical instrument etc. In some embodiments, the surgical robotic system 10 may comprise a microscope which may be communicatively coupled to the surgical robotic system 10.

The surgical robotic system 10 may further comprise a processor subsystem 40 configured for controlling the actuator subsystem 60 to control a position of the surgical instrument 250 during the intraocular procedure. To control the actuator subsystem 60, the processor subsystem 40 may provide actuation commands 42 to the actuator subsystem. For example, in an epiretinal membrane peeling procedure, through such control, the surgical instrument 250 may be positioned relative to the surface of the retina to grasp and peel the epiretinal membrane starting from an edge of the epiretinal membrane. Another example is that during cataract surgery, the surgical instrument 250 may be positioned relative to the surface of the lens or the capsular bag. Yet another example is that during glaucoma surgery, the surgical instrument 250 may be positioned relative to the surface of the trabecular meshwork.

Fig. 2A shows structures of the eye 100 including an eyeball 102, an eye socket 104, a cornea surface layer 106 (also referred to as 'cornea' in short), a sclera surface layer 107 (also referred to as 'sclera' in short), and a lens 108. Fig. 2A shows the eyeball 102 in a neutral orientation, looking substantially straight ahead and the eyeball 102 substantially centrally aligned within the eye socket 104. To be able to see a posterior region of the eye, for example to obtain visual feedback during a surgery in the posterior region of the eye, an observer may view the posterior region through the cornea 106 and the lens 108, for example using an observation apparatus such as a microscope. Fig. 2A schematically shows the field of view 110 in the posterior region when the eye 100 is observed from a straight-ahead perspective. The field of view 110 is thereby limited to a central region of the posterior region of the eye 100 and does not reach far into the peripheral posterior region of the eye 100.

To address this deficiency in observability, orbital manipulation may be performed, by which the eyeball 102 may be rotated within the eye socket 104. For example, a surgeon may rotate the eye using two instruments that are inserted into the eye (not shown in Fig. 2A). **Fig. 2B** shows a result of such orbital manipulation, showing the eyeball 102 having been rotated sideways within the eye socket 104. From the perspective of an observer or observation apparatus viewing the eye 100 from a straight-ahead perspective, the field of view 112, which is shown in a schematic manner in Fig. 2B, may now include a peripheral posterior region of the eye 100. For reference, the field of view 110 of the central posterior region of the eye 100 of Fig. 2A remains shown in Fig. 2B. The orbital manipulation may not only improve the visibility of the peripheral posterior region of the eye 100, but in some cases may also improve the reach of a surgical instrument and/or may allow certain types of tissue to be pushed within the field of view 112, for example by indentation of the sclera.

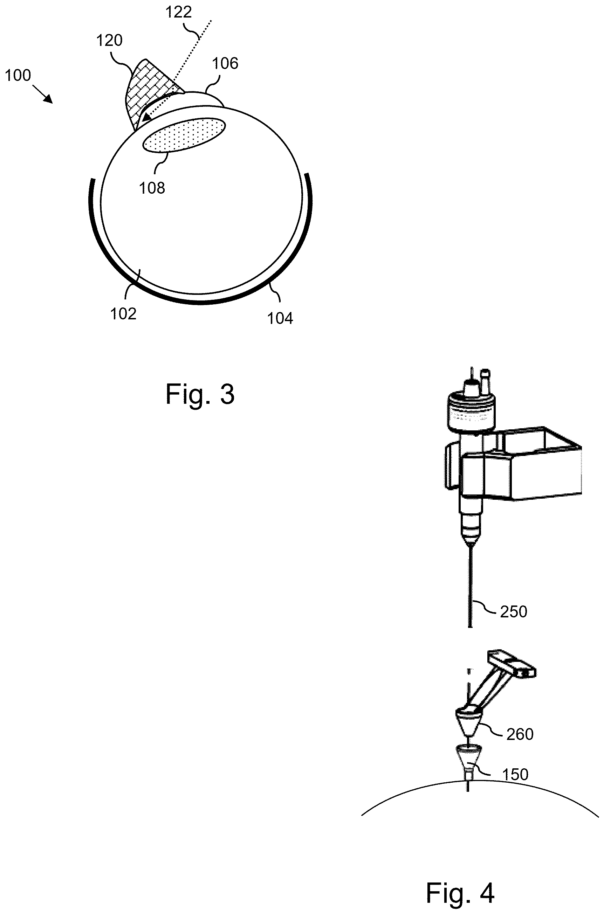

Fig. 3 shows a sideways orientation of the eye 100 which may be obtained by orbital manipulation. At the sideways orientation of the eye 100, anatomical structures in the far periphery of the anterior chamber of the eye may be observed. For example, when observing the eye along a view axis 122 and when using a gonio prism 120, the trabecular meshwork may be observed during a gonioscopy procedure.

Figs. 4-8 describe a surgical robotic system and aspects of the operation of the surgical robotic system. The surgical robotic system may for example be of a general type as previously described with reference to Fig. 1, in that aspects previously described with reference to Fig. 1 may equally apply to the presently described surgical robotic system. The presently described surgical robotic system may be configured to perform orbital manipulation of the eye rather than, for example, relying on an external system or a surgeon to perform the orbital manipulation. For that purpose, the described surgical robotic system may be configured to engage the eye and apply force to the eye to reorient the eye within the eye socket. Specifically, as shown in **Fig. 4****,** the surgical robotic system may comprise a cannula interface 260 that is configured to releasably engage a cannula 150 inserted into the eye at the entry point. Such a cannula 150 may enable a surgical instrument, held manually by a surgeon or by the aforementioned surgical robotic system, to enter the eye. The surgical robotic system may be configured to at least in part effect the orbital manipulation of the eye by applying force through the cannula interface 260 to the cannula 150, which in turn may transmit the force to the eye to facilitate controlled rotation of the eye.

Although not explicitly shown in Fig. 4, the cannula interface 260 may be mechanically linked to the instrument manipulator. As a result, movement of the surgical instrument 250, which is held by the instrument manipulator, may be coupled to movement of the cannula interface 260, allowing coordinated control of the surgical instrument 250 and the cannula interface 260 during orbital manipulation of the eye.

Although not described with reference to Figs. 4-8, force may alternatively be applied in other ways to effect orbital manipulation. For example, the processor subsystem may apply force directly with the inserted surgical instrument to the cannula, or may apply force with the inserted surgical instrument directly to the eye, for example to the cornea or the sclera adjacent to the entry point. In other embodiments, orbital manipulation of the eye may be performed using a separate eye gripping device held by the surgical robotic system, such as a vacuum cup or an eye gripping device equipped with a needle bed. In yet other embodiments, orbital manipulation of the eye may be performed by an external system, such as a separate eye manipulation system. In such cases, the surgical robotic system may obtain data characterizing the orbital manipulation of the eye, also referred to as eye manipulation data, from the external system or from a sensor configured to observe the orbital manipulation of the eye.

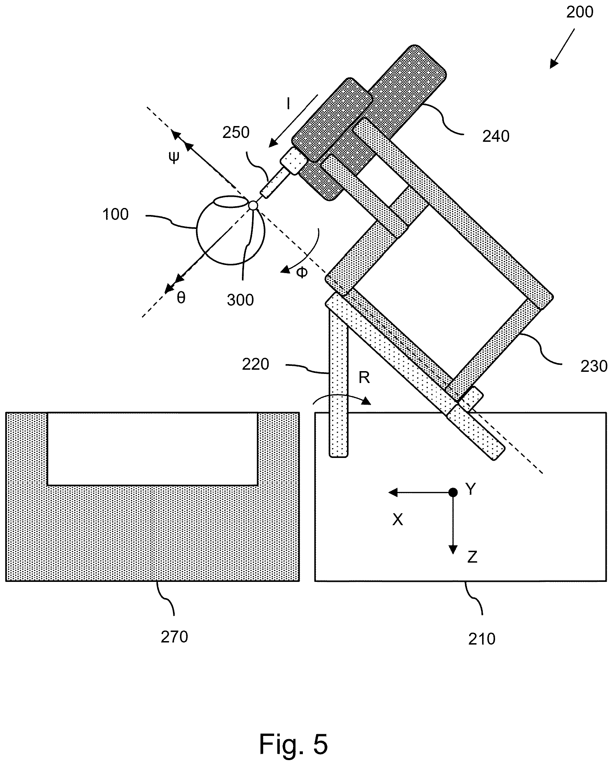

**Fig. 5** shows a surgical robotic system 200 which comprises an instrument manipulator 240 for mounting a surgical instrument 250. As such, the instrument manipulator 240 may also be referred to as a 'mount' for the surgical instrument 250. Another term for instrument manipulator may be 'manipulator head'. The surgical instrument 250 may be a tool or device for performing specific actions or carrying out desired effects during a surgery or operation, such as modifying biological tissues or providing access for viewing biological tissue. Examples of surgical instruments include, but are not limited to, forceps, mechanical cutters, coagulation cutters, scissors, injection needles, sealing devices, etc. The instrument manipulator 240 may be movably suspended by a linkage system 230. The linkage system 230 may for example be a parallelogram linkage system for positioning the instrument manipulator. The instrument manipulator 240 may be suspended on a suspension point of the instrument manipulator via a linkage component coupled to the instrument manipulator. By way of the linkage system 230, or by movably suspending the instrument manipulator in another manner, the instrument manipulator 240 may be provided with at least one degree of freedom to enable adjustment of a pose of the surgical instrument 250. For example, the surgical instrument 250 may be moved longitudinally in the direction I.

Fig. 5 further shows the surgical robotic system 200 to comprise a hold-and-release mechanism 220 arranged for holding the linkage system 230 and thereby holding the instrument manipulator 240 at a surgical position proximate to a patient and for releasing ('R' in Fig. 5) the linkage system 230 and thereby releasing the instrument manipulator 240 from the surgical position to a standby position distal to the patient. The hold-and-release mechanism 220 may be mounted to a base 210 of the surgical robotic system 200, which base 210 may be movable in 3D physical space, for example in a 3D Cartesian coordinate system, e.g., in X, Y, Z, or in a 3D polar coordinate system. Fig. 5 further shows a headrest 270 for holding a head of the patient. In some examples, the headrest 270 may be separate from the surgical robotic system 200 but may have a known physical position relative to the base 210 of the surgical robotic system 200. In other examples, the headrest 270 may be connected to the base 210 of the surgical robotic system 200, for example in a mechanical manner.

Although not separately shown in Fig. 5, the surgical robotic system 200 may comprise an actuation subsystem and a processor subsystem which may be jointly arranged to effect the adjustment of the pose of the surgical instrument 250. The actuation subsystem may comprise one or more actuators and the processor subsystem may control the actuator(s). For example, the actuation subsystem may actuate one or more of the base 210, the hold-and-release mechanism 220, the linkage system 230, and the instrument manipulator 240. For example, the actuation subsystem may comprise one or more actuators, and the processor subsystem may control the actuators accordingly. In a specific example, the actuators may comprise servo motors, stepper motors, or pneumatic cylinders, which provide controlled movement. The actuators may be controlled by the processor subsystem using various methods such as pulse-width modulation (PWM), digital signals, or analog control voltages. The processor subsystem may use feedback mechanisms such as encoders, potentiometers, or linear variable differential transformers (LVDTs) to monitor and adjust the position and speed of the actuators. Optionally, the processor subsystem may implement control strategies such as PID (Proportional-Integral-Derivative) control, feedforward control, or adaptive control. Using the actuation subsystem, the surgical instrument 250 may be adjusted in pose to have a variable remote center of motion (RCM) 300 on a surface of the eye 100. For example, the surgical instrument 250 may be adjusted in pose in I and Θ directions by actuation of the instrument manipulator 240, and in Φ direction and Ψ direction by actuation of the linkage system 230.

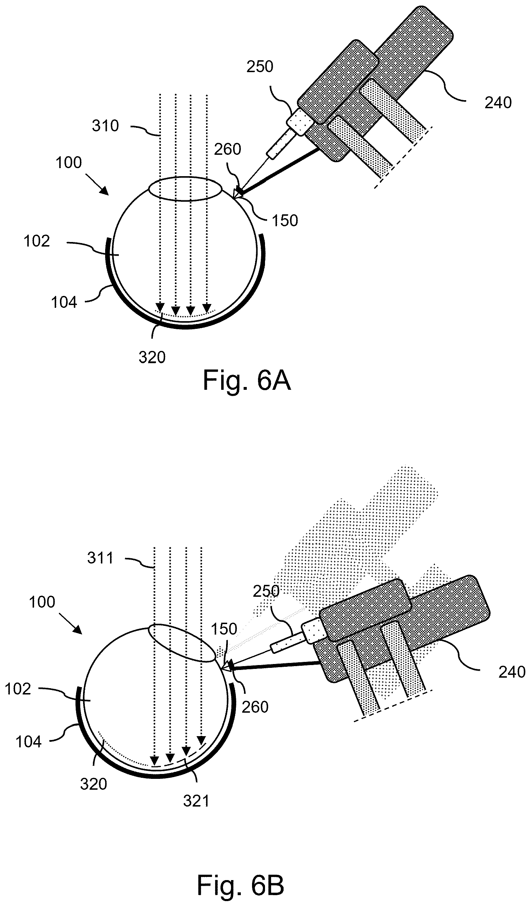

Figs. 6A and 6B schematically illustrate orbital manipulation of the eye by the surgical robotic system of Fig. 5 using the cannula interface 260, wherein both the cannula interface 260 and the RCM (not shown explicitly) of the surgical instrument 250 are repositioned simultaneously. **Fig. 6A** shows an initial orientation in which the instrument manipulator 240 is positioned such that the cannula interface 260 engages the cannula 150 inserted into the eye 100. The surgical instrument 250 may or may not be inserted into the eye 100. In this orientation, the eyeball 102 is positioned within the eye socket 104 such that a posterior region 320 of the retina is visible within a field of view 310 of for example an observer or an observation apparatus such as a microscope. In some cases, it may be desirable to perform orbital manipulation to enable observation of a different posterior region of the retina. For this purpose, the surgical robotic system may reorient the eyeball 102 within the eye socket 104. Fig. 6B illustrates the result of such reorientation, showing a reorientation of the eyeball 102 within the eye socket 104 that allows a different posterior region 321 of the retina to be observed. To achieve this reorientation of the eyeball 102, the processor subsystem of the surgical robotic system may adjust the pose of the instrument manipulator 240 so that the distal end of the cannula interface 260, which engages the cannula 150, moves along the curvature of the eye 100, thereby inducing the repositioning of the eyeball.

Preferably, the pose of the instrument manipulator 240 may be adjusted such that the surgical instrument 250 is maintained at a substantially constant tilt angle with respect to the entry point during orbital manipulation. For example, the surgical instrument 250 may be maintained substantially perpendicular to the ocular surface at the entry point. In this context, 'substantially perpendicular' may for example refer to a tilt angle within ±20%, ±10%, or ±5% of a perfectly perpendicular orientation.

With continued reference to the repositioning of the RCM to track the entry point, it is noted that orbital manipulation of the eye may be performed by an external system or in a different manner by the surgical robotic system. As such, the following considerations apply to the repositioning of the RCM to track the entry point, irrespective of the entity performing the orbital manipulation or the manner in which it is carried out, e.g., with a cannula interface or otherwise. To reposition the RCM, the processor subsystem of the surgical robotic system may access a model indicative of the repositioning of the entry point in three-dimensional space during orbital manipulation of the eye and reposition the RCM in accordance with the model during the orbital manipulation. Here, 'in accordance with the model' may refer to a repositioning which takes the outer surface curvature of the eye into account.

The model may, for example, be a spherical model or an ellipsoidal model of the eye. In some cases, the model may be generated or adapted based on preoperative or intraoperative sensor data to obtain a patient-specific model. For example, the model may be fitted to data points obtained from sensor data, and the fitted model may then be used to control the repositioning of the RCM.

Such sensor data may be acquired intraoperatively during the ophthalmic procedure or preoperatively. For example, extraocular sensor data may be acquired by one or more sensors positioned outside the eye. Such sensor data may provide information about the curvature of the eye, for instance, by measuring the ocular surface externally or by generating a cross-sectional profile of the eye, as in the case of optical coherence tomography (OCT). Additionally, or alternatively, the processor subsystem may utilize intraocular sensor data, which may be obtained from a sensor affixed to the surgical instrument. For example, an optical fiber may be attached to the surgical instrument and connected to an OCT system located outside of the eye. Such intraoperative sensor data may indicate the distance between the inserted surgical instrument and the retinal surface. This distance measurement may provide information about the patient-specific shape of the eye from within the eye, which may in turn serve as a predictor of the outer surface curvature of the eye.

In general, data points on the outer surface of the eye or the inner retinal surface may be used for model fitting. Sphere fitting, referring to the fitting of data points to a parameterized spherical model, may be explained as follows. A sphere may be parameterized by its center point (x₀, y₀, z₀) and its radius *r*. An online sphere fitting problem can be derived from a general sphere equation as $arg {min}_{\left({x}_{0}, {y}_{0}, {z}_{0}, r\right)} \left‖\left(\begin{matrix}{x}_{i}^{2} \\ {y}_{i}^{2} \\ {z}_{i}^{2} \\ 1\end{matrix}\right)⋅\left(\begin{matrix}2 {x}_{0} \\ 2 {y}_{0} \\ 2 {z}_{0} \\ {r}^{2} - {x}_{0}^{2} - {y}_{0}^{2} - {z}_{0}^{2}\end{matrix}\right)-\left({x}_{i}^{2}+{y}_{i}^{2}+{z}_{i}^{2}\right)\right‖ ,$ to obtain linear dependencies between a given measurement point (*xᵢ, yᵢ, zᵢ*), and parameters (*x*₀, *y*₀*, z*₀, *r*) to be optimized. Here, the term 'online fitting' may refer to the modelling of the surgical environment not being available at the beginning of the surgical procedure but being acquired progressively by acquiring measurement points during the procedure and the model being continuously updated to better fit the acquired measurement points. To solve this online sphere fitting problem, a Kalman filter or any other suitable optimization technique may be used.

Ellipsoid fitting, referring to the fitting of data points to a parameterized ellipsoid model, may be explained as follows. An ellipsoid may be parameterized by its center point (*x*₀, *y*₀, *z*₀), semi-axes (*r₁*, *r*₂, *r*₃) and orientation (*α, β, y*)*.* For example, the residual of the general quadratic equation ${ax}^{2} + {by}^{2} + {cz}^{2} + 2 fyz + 2 gxz + 2 hxy + 2 px + 2 qy + 2 rz + d = 0$ may be minimized to obtain linear dependencies between the given measurement point (*xᵢ, yᵢ, zᵢ*) and the parameters (*a, b, c, f, g, h, p, q, r, d*)*,̅* and to avoid nonlinear optimization. An additional nonlinear constraint $4 \left(ab+bc+ac-{f}^{2}-{g}^{2}-{h}^{2}\right) - {\left(a+b+c\right)}^{2} > 0$ may be used to specifically fit ellipsoids. The Kalman filter may be extended to deal with such nonlinear constraints. Another option is to use Cayley Transform Ellipsoid Fitting (CTEF) to find the best fit parameters.

With continued reference to the repositioning of the remote center of motion (RCM) to track the entry point, it is noted that the processor subsystem may, during the repositioning of the RCM, obtain intraoperative sensor data and analyze the intraoperative sensor data to detect a deviation of the RCM from the entry point and/or detect a deviation of the RCM from the ocular surface of the eye. Such a deviation may indicate that the model does not accurately represent the eye or may result from other inaccuracies, such as errors in the control of the RCM.

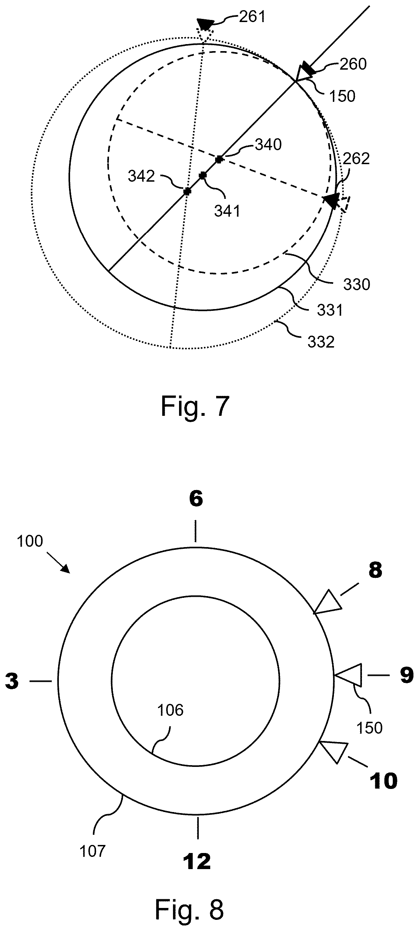

**Fig. 7** illustrates the consequences of using inaccurate models. The figure depicts a model 331 that accurately represents the surface of the eye (albeit shown in a simplified form as a spherical model), a model 330 that underestimates the radius of the eye, and a model 332 that overestimates the radius of the eye. As a result of the different modeling of the radius of the eye, the center of rotation differs among the models, as all models may be aligned with the observable ocular surface. Specifically, the center of rotation 340 of model 330 is positioned too close to the ocular surface, while the center of rotation 342 of model 332 is positioned too deep relative to the ocular surface, compared to the correct center of rotation 341 of model 331.

Fig. 7 further illustrates the consequences of using each model for repositioning the RCM. Here, it is assumed that the RCM is co-located with the cannula interface. If model 331 is used, the cannula interface 260 may maintain engagement with the cannula 150. However, if model 330, which underestimates the radius of the eye, is used, the surgical robotic system may press the cannula interface 262 into the eye, causing the cannula interface 262 to indent the ocular surface (symbolically shown in Fig. 7 by the cannula interface 262 having moved past the cannula). Conversely, if model 332, which overestimates the radius of the eye, is used, the cannula interface 261 may lose engagement with the cannula. If the cannula interface 261 is also used for orbital manipulation, such loss of engagement may reduce the effectiveness of the orbital manipulation or cause the orbital manipulation to cease altogether.

To compensate for deviation of the RCM from the entry point and/or the ocular surface of the eye, the processor subsystem may control the actuator to adjust the position of the RCM based on the detected deviation, for example by, as needed, retracting the RCM away from or advancing the RCM toward the ocular surface or by adjusting the lateral position of the RCM relative to the lateral position of the entry point on the ocular surface to reduce the deviation.

The processor subsystem may detect the deviation of the RCM from the ocular surface using intraoperative sensor data, which may include various types of sensor inputs acquired during surgery. In some embodiments, the intraoperative sensor data includes image data of the entry point, for example image data acquired using a microscope. The processor subsystem may analyze the image data to detect a deviation of the RCM from the entry point and/or the ocular surface. For example, the processor subsystem may analyze the image data to determine whether an indentation of the eye has occurred due to contact with the surgical robotic system and, if so, may retract the RCM accordingly. Likewise, if the image data indicates that a distal part of the surgical robotic system is moving away from the ocular surface, the processor subsystem may advance the RCM toward the ocular surface to compensate for the deviation. Another example is that the image data may indicate that the surgical robotic system has, with its cannula interface, lost connection with the cannula at the entry point. In response, the processor subsystem may adjust the position of the RCM, which is typically co-located with the cannula interface. For example, the processor subsystem may advance the RCM toward the ocular surface to re-establish contact, retract the RCM away from the ocular surface if excessive force or indentation is detected, and/or adjust the lateral position of the RCM relative to the ocular surface to compensate for misalignment. By dynamically modifying the RCM's position in three-dimensional space, the processor subsystem may restore and maintain proper engagement between the cannula interface and the cannula at the entry point.

In some embodiments, the intraoperative sensor data includes intraocular pressure (IOP) data obtained from an intraocular pressure sensor. The processor subsystem may monitor changes in intraocular pressure and adjust the position of the RCM based on detected variations in the measured pressure. Intraocular pressure is typically stabilized during vitrectomy through an infusion line connecting the eye to a vitrectomy machine. Since maintaining a stable IOP is desirable, deviations in IOP may indicate an incorrect rotation trajectory or excessive force exerted on the eye. For example, an increase in IOP may suggest that the eye is being indented due to excessive force, which may require an increase in the radius of rotation or retraction of the RCM away from the ocular surface to mitigate further pressure buildup. Conversely, a decrease in IOP may indicate a loosening of the cannula connection, requiring a reduction in the radius of rotation or repositioning of the RCM to restore engagement.

In some embodiments, the intraoperative sensor data includes force sensor data indicative of a force exerted on the eye by the surgical robotic system. The processor subsystem may adjust the position of the RCM based on variations in the measured force. The force data may be obtained from a force sensor attached to an instrument manipulator. In some implementations, the force sensor may be configured to measure a force exerted by the cannula interface attached to the instrument manipulator on a cannula inserted into the eye. Additionally, or alternatively, the force sensor may measure a force exerted by the inserted surgical instrument on the cannula or directly on the eye. Examples of force sensors include, but are not limited to, fiber-optic, MEMS-based, and piezoelectric sensors. The force sensor may be used to monitor axial forces during rotation. The processor subsystem may use the measured force data to determine whether adjustments to the position of the RCM are needed to maintain a desired level of force interaction between the surgical robotic system and the ocular surface. In some implementations, the processor subsystem may control the rotation trajectory of the eye to maintain a constant force, ensuring that forces exerted on the eye remain within acceptable limits while the RCM is repositioned.

In some implementations, the processor subsystem may adjust the position of the RCM based on the intraoperative sensor data using specific threshold values. For example, the processor subsystem may be configured to retract the RCM away from the ocular surface if a measured parameter of the intraoperative sensor data exceeds an upper threshold or if an increase in the measured parameter exceeds an upper change threshold. Conversely, the processor subsystem may advance the RCM toward the ocular surface if the measured parameter falls below a lower threshold or if a decrease in the measured parameter exceeds a lower change threshold.

In some embodiments, the intraoperative sensor data includes distance data obtained from an instrument-attached distance sensor, such as an OCT probe. The processor subsystem may analyze the distance data and adjust the position of the RCM based on detected changes in the measured distance. While the measured distance may correspond to an internal distance within the eye, it may also provide an indication of the distance between the RCM and the ocular surface. This is because the processor subsystem may adjust the pose of the surgical instrument during the repositioning of the RCM to maintain a constant relative pose of the surgical instrument with respect to the retinal surface of the eye. If the measured distance to the retinal surface changes, this may indicate that the repositioning of the RCM is not correctly following the contour of the ocular surface. For example, if the distance sensor detects an increase in distance between the surgical instrument and the retinal surface, the processor subsystem may advance the RCM toward the ocular surface to restore the relative positioning between the surgical instrument and the retinal surface. Conversely, if the distance sensor detects a decrease in distance to the retinal surface, the processor subsystem may retract the RCM away from the ocular surface.

In some embodiments, the processor subsystem may be configured to analyze intraoperative sensor data to detect a deviation between a modeled representation of the ocular surface of the eye and the actual ocular surface of the eye. Based on the detected deviation, the processor subsystem may update the modeled representation to improve accuracy. For example, if the detected deviation indicates a discrepancy between the estimated and actual ocular surface, the processor subsystem may adjust certain parameters of the model, such as an estimate of the radius of the eye. The adjustment may be performed in small stepwise increments, allowing the model to be refined progressively through repeated updates, thereby optimizing the representation of the ocular surface over time.

In a specific example, the deviation between the RCM and the ocular surface may be directly used as feedback to update the model. For example, if the detected deviation indicates that the RCM is moving away from the ocular surface, this may suggest that the model overestimates the radius of the eye, prompting the processor subsystem to reduce the modeled radius accordingly. Conversely, if the deviation indicates that the RCM is moving toward the ocular surface, this may suggest that the model underestimates the radius of the eye, leading the processor subsystem to increase the modeled radius to better align with the actual ocular surface.

**Fig. 8** shows a top-down anterior view of eye 100, illustrating the cornea 106, the sclera 107, and various possible placements of a cannula 150 relative to a clock face reference. The figure depicts different angular placements of the cannula around the eye (e.g., different "hour" positions). However, the cannula placement may also vary in its radial distance from the center of the eye. For example, even for a placement at 9 o'clock, the cannula may be positioned at different distances from the limbus, typically ranging from approximately 3 to 4 mm. Such variations in both angular and radial placement may be relevant when considering the trajectory and alignment of the surgical instrument. Accordingly, the placement of the cannula, or more generally the position of the entry point on the eye, may be taken into account by the surgical robotic system when using the model to reposition the RCM. The surgical robotic system may be initially positioned with its RCM aligned with the entry point on the eye, for example as a result of a preparatory setup procedure in which the surgical robotic system and instruments are positioned before the actual intraocular surgery commences. The processor subsystem may therefore assume that the initial position of the entry point on the ocular surface corresponds to the initial position of the RCM relative to the ocular surface. This initial positioning of the RCM relative to the ocular surface may be used to initialize the application of the model, allowing the processor subsystem to relate the real-world positioning of the RCM to the modeled representation. This may be particularly relevant when the model used by the processor subsystem deviates from a perfectly spherical representation of the eye, as an accurate reference point enables more precise adjustments during the repositioning of the RCM. The placement of the cannula may be determined from extraocular sensor data, such as image data obtained from a microscope or an external camera, or may be received as user input.

Additionally, or alternatively, to using the position of the entry point on the eye to initialize the application of the model, the initial orientation of the eye relative to the instrument manipulator may be taken into account by the surgical robotic system when using the model to reposition the RCM. For that purpose, the surgical robotic system may obtain data indicative of the eye's initial orientation prior to orbital manipulation. This data may be obtained from extraocular sensor data, such as imaging or tracking data, or may be provided as user input. The processor subsystem may use the initial orientation as a reference when applying the model for repositioning the RCM, ensuring that adjustments remain consistent with the actual positioning of the eye. This may be particularly relevant when the model used by the processor subsystem deviates from a perfectly spherical representation of the eye.

In some embodiments, the surgical robotic system may be configured to perform orbital manipulation with the cannula interface, and the processor subsystem may control the pose of the instrument manipulator so that the surgical instrument remains substantially perpendicular to the ocular surface during the orbital manipulation. For that purpose, an assumed rotation trajectory may be determined based on parameters such as an estimated eye size, a cannula placement location, and an initial eye orientation where the eye is facing straight upward. In the case of an idealized spherical eye model with a center of rotation located at the geometric center of the sphere, a corresponding ideal rotation trajectory may be predefined. By moving the cannula interface along this trajectory, the eye may be rotated through the mechanical connection between the cannula interface and the cannula. Simultaneously, the processor subsystem may control the surgical instrument orientation such that surgical instrument remains directed toward the center of the eye, which may ensure that the instrument remains within the surgeon's field of view and away from the retinal surface to avoid damages.

To facilitate orbital manipulation, the eye may need to be secured or stabilized at a specific gripping point. The manner in which the eye is gripped may be achieved in various ways, including by exerting lateral force on the eye through an instrument inserted into the cannula, as is commonly done in manual eye rotation, or by using a dedicated eye gripping device. Alternatively, the existing mechanical connection between the cannula and a specialized robotic cannula interface may be utilized. A combination of these approaches may also be employed, for example, using both the cannula connection and an instrument inserted through the cannula.

When rotating the eye, it may be important to ensure that the gripping point follows a natural rotation trajectory to prevent excessive forces on the eye, which could cause damage or result in loss of the cannula connection. If the gripping point does not follow the appropriate trajectory, unwanted forces may be exerted, potentially leading to ocular deformation or detachment of the cannula connection, which in turn could lead to a loss of control over eye rotation. A challenge in implementing controlled eye rotation is the determination of the correct trajectory. If the assumed trajectory does not match the actual curvature of the eye, excessive force may be applied, potentially causing eye indentation or loss of the cannula connection. Several factors contribute to this complexity, including individual variations in eye size and shape, differences in the center of rotation between horizontal and vertical eye movements, and variations in cannula placement and initial eye orientation at the time of starting orbital manipulation.

Various methods may be employed to determine the correct trajectory before orbital manipulation. This may generally involve using a model that represents the repositioning of the entry point in three-dimensional space during orbital manipulation of the eye. One approach involves calculating the center of rotation based on preoperative data. Parameters such as eye shape and axial length, obtained from preoperative imaging, may be used to generate a patient-specific estimate of the center of rotation and the corresponding rotation trajectory, which may serve as a model of the eye. Additionally, intraoperative imaging data from a surgical microscope (OPMI) may be used to refine the estimated trajectory by incorporating real-time information, such as the exact placement of the cannula and the initial orientation of the eye.

Another approach involves utilizing intraoperative and instrument-integrated optical coherence tomography data to provide real-time distance measurements from the instrument tip to the retina. This may be achieved by incorporating an optical fiber into the instrument and using an OCT engine to perform distance measurements. Prior to initiating orbital manipulations, these distance measurements may be used to assess various ocular parameters, such as axial length in different directions, thereby refining the model and thus the calculated trajectory.

It should be noted that the above-mentioned embodiments illustrate rather than limit the presently disclosed subject matter, and that those skilled in the art will be able to design many alternative embodiments without departing from the scope of the appended claims.

In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The mere fact that certain measures are recited in mutually different dependent claims does not indicate that a combination of these measures cannot be used to advantage.

## Claims

1. A surgical robotic system (10, 200) for ophthalmic surgery, comprising:
- an instrument manipulator (240) for mounting a surgical instrument (250), wherein the surgical instrument comprises an intraocular portion for insertion into an eye (100) via an entry point on an ocular surface of the eye, wherein the instrument manipulator comprises at least one degree of freedom to enable adjustment of a pose of the surgical instrument;
- an actuation subsystem (60) configured to actuate the adjustment of the pose of the surgical instrument; and
- a processor subsystem (40) configured to control the actuation subsystem to control the adjustment of the pose of the surgical instrument;
wherein the surgical robotic system is configured to establish a remote center of motion (300), RCM, for the surgical instrument and to control a position of the RCM in three-dimensional space;
wherein the processor subsystem is configured to reposition the RCM to track the entry point during orbital manipulation of the eye by:
- accessing a model (330-332) indicative of a repositioning of the entry point in three-dimensional space during orbital manipulation of the eye;
- repositioning the RCM in accordance with the model during the orbital manipulation of the eye;
- obtaining intraoperative sensor data (32) and analyzing the intraoperative sensor data to detect a deviation of the RCM from the entry point and/or the ocular surface of the eye; and
- adjusting the position of the RCM based on the detected deviation.

2. The surgical robotic system (10, 200) according to claim 1, wherein the surgical robotic system is configured to effect the orbital manipulation of the eye (100) by engaging and applying a force to the eye to reorient the eye within an eye socket (104).

3. The surgical robotic system (10, 200) according to claim 2, wherein the instrument manipulator (240) comprises a cannula interface (260-262) configured to releasably engage a cannula (150) inserted into the eye at the entry point, and wherein the processor subsystem (40) is configured to at least in part effect the orbital manipulation of the eye (100) by applying the force with the cannula interface to the cannula.

4. The surgical robotic system (10, 200) according to claim 2, wherein the surgical instrument (250) is inserted through a cannula (150) into the eye, wherein the processor subsystem (40) is configured to at least in part effect the orbital manipulation of the eye (100) by applying the force with the inserted instrument to the cannula.

5. The surgical robotic system (10, 200) according to claim 2, wherein the processor subsystem (40) is configured to at least in part effect the orbital manipulation of the eye (100) by applying the force with the inserted instrument (250) directly to the eye, for example to the cornea (106) or sclera (107) adjacent to the entry point.

6. The surgical robotic system (10, 200) according to any one of claims 1 to 5, wherein the model (300-332) is configured to provide a modeled representation of the ocular surface of the eye (100) and to adapt the model to a patient-specific shape of the eye based on an estimate of an ocular radius of the eye.

7. The surgical robotic system (10, 200) according to claim 6, wherein the model is a parameterized geometric model, and wherein the processor subsystem (40) is configured to determine a center of rotation (340-342) and/or spatial dimensions of the geometric model based on the estimate of the ocular radius of the eye (100).

8. The surgical robotic system (10, 200) according to claim 7, wherein the processor subsystem (40) is configured to:
- obtain a distance measurement from a distance sensor, wherein the distance data is indicative of a distance between the surgical instrument and an ocular structure;
- adjust the pose of the surgical instrument to obtain a plurality of distance measurements from the distance sensor; and
- update the model (330-332) based on the plurality of distance measurements.

9. The surgical robotic system (10, 200) according to any one of claims 1 to 8, wherein the processor subsystem (40) is configured to update the model (330-332) based on the detected deviation of the RCM (300) from the entry point and/or the ocular surface of the eye.

10. The surgical robotic system (10, 200) according to any one of claims 1 to 9, wherein the intraoperative sensor data (32) comprises image data of the entry point, for example acquired using a microscope, wherein the processor subsystem (40) is configured to analyze the image data to detect a deviation of the RCM (300) from the entry point and/or the ocular surface, for example by detecting an indentation of the eye (100) caused by the surgical robotic system.

11. The surgical robotic system (10, 200) according to any one of claims 1 to 10, wherein the intraoperative sensor data (32) comprises intraocular pressure data acquired from an intraocular pressure sensor (30), wherein the processor subsystem (40) is configured to adjust the position of the RCM (300) based on a change in measured intraocular pressure.

12. The surgical robotic system (10, 200) according to any one of claims 1 to 11, wherein the intraoperative sensor data (32) comprises force sensor data indicative of a force exerted on the eye by the surgical robotic system, wherein the processor subsystem (40) is configured to adjust the position of the RCM (300) based on a change in measured force.

13. The surgical robotic system (10, 200) according to claim 12, wherein the force data (32) is obtained from a force sensor attached to the instrument manipulator (240), wherein the force sensor is configured to measure a force exerted by a cannula interface (260-262) attached to the instrument manipulator to a cannula (150) inserted into the eye (100) and/or a force exerted by the inserted surgical instrument (250) to the cannula or directly to the eye.

14. The surgical robotic system (10, 200) according to any one of claims 1 to 13, wherein the intraoperative sensor data (32) comprises distance data from a distance sensor (30), wherein the processor subsystem (40) is configured to adjust the position of the RCM (300) based on a change in measured distance.

15. The surgical robotic system (10, 200) according to any one of claims 1 to 14, wherein the processor subsystem (40) is configured to adjust the pose of the surgical instrument (250) during the repositioning of the RCM (300) to maintain a constant relative pose of the surgical instrument with respect to a retinal surface of the eye (100).
